(19) Europäisches Patentamt
European Patent Office
Office européen des brevets

(11) Numéro de publication: **0 314 569 B1**

(12) **FASCICULE DE BREVET EUROPEEN**

(45) Date de publication de fascicule du brevet: **02.03.94**

(51) Int. Cl.5: **C12N 15/86, A61K 39/275, A61K 39/155, A61K 39/165, A61K 39/175, A61K 39/295**

(21) Numéro de dépôt: **88402695.6**

(22) Date de dépôt: **26.10.88**

(54) **Virus du fowlpox recombinant, vecteurs d'expression de protéines héterologues et vaccins pour la volaille dérivés de ce virus.**

(30) Priorité: **29.10.87 FR 8715001**
**20.06.88 FR 8808226**

(43) Date de publication de la demande:
**03.05.89 Bulletin 89/18**

(45) Mention de la délivrance du brevet:
**02.03.94 Bulletin 94/09**

(84) Etats contractants désignés:
**AT BE CH DE ES FR GB GR IT LI LU NL SE**

(56) Documents cités:
**EP-A- 0 227 414      EP-A- 0 257 994
EP-A- 0 284 416      WO-A-86/00528
WO-A-86/05806      WO-A-88/02022
US-A- 3 429 965**

**JOURNAL GEN VIROL, vol. 67, 1986, GB; D.B. BOYLE et al., pp. 1591-1600&NUM;**

**VIROLOGY, vol. 160, no. 1, 1987; R. DRILLIEN et al., pp. 203-209&NUM;**

(73) Titulaire: **TRANSGENE S.A.**
**11, rue de Molsheim**
**F-67000 Strasbourg(FR)**

(72) Inventeur: **Drillien, Robert**
**10, Bd Paul Déroulède**
**F-67100 Strasbourg(FR)**
Inventeur: **Spehner, Danièle**
**29, rue de la Chênaie**
**F-67200 Eckbolsheim(FR)**

(74) Mandataire: **Warcoin, Jacques et al**
**Cabinet Régimbeau**
**26, avenue Kléber**
**F-75116 Paris (FR)**

EP 0 314 569 B1

VACCINE, vol. 5, no. 1, mars 1987, pages 65-70, Butterworth & Co. (Publishers) Ltd., Guildford, Surrey (GB); M. MORITA et al., pp. 65-70&NUM;

NATURE, vol. 326, no. 6116, 30 avril-06 mai 1987, Londres (GB); R. LATHE et al., pp. 878-880&NUM;

CHEMICAL ABSTRACTS, vol. 103, no. 1, juillet 1985, Columbus, OH (US); S.E.H. RUSSELL et al., pp. 152-153, no. 1555s&NUM;

GENE, vol. 47, no. 2/3, 1986, Elsevier Science Publishers B.V., Amsterdam (NL); D. PANICALI et al., pp. 193-199&NUM;

BIOLOGICAL ABSTRACTS, vol. 10, no. 4, 1988, Philadelphia, PA (US); D.B. BOYLE et al., pp. 343-356&NUM;

PROCEEDINGS OF THE NATL. ACADEMY OF SCIENCES USA, vol. 85, février 1988, US; R. DRILLIEN et al., pp. 1252-1256&NUM;

**Description**

La présente invention concerne des virus recombinant de la variole de poule, communément appelée Fowlpox, et leurs applications à la préparation de vaccins ou de protéines utiles au métabolisme de la volaille.

Les virus de la famille des Poxviridae, dont le virus du Fowlpox fait partie, sont des virus à ADN double brin qui se multiplient dans le cytoplasme des cellules et codent pour une partie importante de la machinerie enzymatique nécessaire à leur réplication.

L'ADN viral purifié n'est pas infectieux parce que des enzymes associées à la particule virale sont nécessaires pour amorcer son expression et sa réplication. Toutefois, il peut participer à des évènements de recombinaison avec l'ADN d'un virus infectieux introduit dans la même cellule. Cette découverte (Sam et Dumbell, 1981) est la base de la mise au point de vecteurs de clonage et d'expression de gènes étrangers, dérivés du virus de la vaccine (Mackett et al. 1982 ; Panicali et Paoletti, 1982). Des virus de la vaccine recombinants vivants ont permis d'exprimer des antigènes étrangers et, ainsi, d'obtenir des immunisations contre différentes maladies virales ou parasitaires (Smith et al. 1983 ; Panicali et al. 1983 ; Kieny et al. 1984 ; Chakrabarti et al. 1986 ; Hu et al. 1986 ; Kieny et al. 1986).

Bien que des publications récentes aient évoqué l'utilisation du Fowlpox comme vecteur (Brown, 1985 et Taylor et al., 1987), aucun résultat expérimental n'a été publié à ce jour et la biologie moléculaire du virus du Fowlpox n'a pas encore fait l'objet d'études approfondies, à la différence de la biologie du virus de la vaccine qui a été bien étudiée (voir revue de Moss, 1985).

La présente invention a pour objet l'utilisation du virus du Fowlpox à titre de vecteur d'expression de protéines hétérologues.

Plus précisément, l'invention a pour objet un virus du Fowlpox recombinant qui dérive d'une souche atténuée, c'est-à-dire rendue artificiellement non pathogène, et comporte, dans une partie non essentielle de son génome, une séquence d'ADN codant pour tout ou partie d'une protéine hétérologue, ainsi que les éléments assurant son expression par le virus du Fowlpox, dans une cellule appropriée.

Sous un de ses aspects, l'invention vise à préparer un vaccin, en particulier un vaccin qui protège la volaille contre l'une au moins des maladies virales qui peuvent l'affecter. Dans ce cas, la protéine hétérologue est une protéine induisant une réponse immunitaire contre un virus hétérologue. Parmi les virus hétérologues intéressants, on peut citer le virus de la maladie de Newcastle, le virus de la bronchite infectieuse et le virus de la maladie de Marek.

La présente invention concerne, en particulier l'utilisation du virus du Fowlpox pour la préparation de vaccins contre les Morbillivirus et a donc pour objet un virus du Fowlpox, caractérisé en ce que la séquence d'ADN code pour une protéine induisant une réponse immunitaire contre un Morbillivirus.

Parmi les virus appartenant à la famille des Morbillivirus, on peut citer le virus de la rougeole, le virus de la maladie de Carré, le virus de la peste bovine et le virus de la peste de petits ruminants.

L'étude des différents Morbillivirus a permis de mettre en évidence les relations immunologiques entre les protéines de surface du virus de la rougeole et celles des autres Morbillivirus, ainsi que des homologies de séquences entre les ADN codant pour certaines de ces protéines. Ces analogies permettent d'envisager l'utilisation d'antigènes du virus de la rougeole pour la vaccination contre la maladie de Carré, la peste bovine et la peste des petits ruminants. Il est également possible de prévoir l'utilisation des antigènes de surface provenant de chacun des Morbillivirus en système de vaccination homologue ou hétérologue.

Les protéines de surface telles que la protéine hémagglutinante (HA) et la protéine de fusion (F) sont plus particulièrement intéressantes, mais d'autres protéines de structure telles que la nucléoprotéine (NP) pourraient être utilisées. Les séquences d'ADN correspondantes peuvent coder pour la protéine de longueur totale ou bien pour des fragments suffisants pour induire in vitro la synthèse des anticorps neutralisant le Morbillivirus.

Il est également possible de prévoir la mise en oeuvre d'une séquence d'ADN codant pour plusieurs protéines ou plusieurs fragments de celles-ci.

Sous un autre de ses aspects, l'invention vise à produire in vivo, dans la volaille, un facteur intervenant dans son métabolisme, tel que par exemple, une hormone de croissance ou un facteur inducteur de celle-ci (comme le GRF). Dans ce cas, la protéine hétérologue est ledit facteur et le virus du Fowlpox comporte les éléments assurant son expression in vivo. Pour obtenir cette production, on administre à l'animal un virus du Fowlpox selon l'invention qui contient le gène codant pour ce facteur, le virus étant vivant.

Enfin, l'invention permet la préparation de protéines d'intérêt industriel ou thérapeutique en culture cellulaire, notamment dans des cellules aviaires, in vitro mais également in vivo chez l'animal.

L'expression d'une séquence codant pour une protéine étrangère par un Poxvirus et notamment par le virus du Fowlpox implique nécessairement les trois étapes suivantes :

1) la séquence codante doit être alignée avec un promoteur de poxvirus et être insérée dans un segment non essentiel de l'ADN du virus du Fowlpox cloné dans un plasmide bactérien approprié ;

2) les séquences d'ADN du virus du Fowlpox situées de part et d'autre de la séquence codante doivent permettre des recombinaisons homologues entre le plasmide et le génome viral dans une cellule réceptrice ; une double recombinaison conduit à un transfert de l'insert d'ADN du plasmide dans le génome viral dans lequel il sera propagé et exprimé ;

3) la séquence d'ADN intégrée dans le génome du Fowlpox recombinant doit être exprimée dans une cellule appropriée, supportant la multiplication du virus.

On a indiqué précédemment que l'insertion de l'ADN étranger doit se faire dans une partie non essentielle du génome du virus du Fowlpox, c'est-à-dire une partie qui n'empêche pas sa réplication. On aurait pu envisager, comme dans les virus de la vaccine recombinants, d'introduire le gène étranger dans le gène TK. Toutefois, comme la souche de départ est une souche atténuée, on peut craindre que l'altération d'une fonction utile à sa réplication ne constitue un handicap trop important et ne diminue fortement son pouvoir immunogène et, par là, celui de l'antigène étranger exprimé par le virus recombinant. C'est pourquoi, suivant une caractéristique de la présente invention, la séquence d'ADN étranger est introduite dans une zone intergénique non codante du virus du Fowlpox. Cette zone intergénique est de préférence, située entre les ORF 7 et 9 du fragment HindIII de 1.3 K pb du génome, selon Drillien et al. (1987).

L'invention concerne également les cultures de cellules eucaryotes que l'on infecte avec le virus recombinant selon l'invention et qui permettent sa réplication. Parmi les cellules envisageables, on peut citer notamment les cellules aviaires.

L'invention a aussi pour objet des vaccins obtenus à partir de ces virus recombinants, sous forme de virus vivants ou inactivés. Les méthodes de préparation des vaccins sont connues de l'homme de métier et ne seront pas rappelées ici.

L'invention concerne aussi des virus recombinants vivants dont l'administration peut permettre la production in vivo, pendant la multiplication virale, d'un facteur utile au métabolisme de la volaille.

Enfin, l'invention concerne des séquences d'ADN comportant au moins un gène hétérologue au virus du Fowlpox, sous le contrôle d'un promoteur de poxvirus, flanqué de part et d'autre de l'extrémité 3′ de l'ORF 7 et de l'extrémité 5′ de l'ORF 9, ainsi que des plasmides portant ces séquences d'ADN.

Ces plasmides sont utiles notamment dans le cadre d'un procédé d'obtention d'un virus recombinant selon l'invention, caractérisé en ce qu'on transfecte une culture de cellules compétentes avec l'ADN d'un plasmide selon l'invention, ladite culture ayant été préalablement infectée par un virus du Fowlpox comportant éventuellement un élément de sélection.

D'autres caractéristiques et avantages de l'invention apparaîtront dans la description détaillée suivante. Les 4 figures suivantes illustrent les exemples :

Figure 1 :    représentation schématique du plasmide pTG1170 et de l'insert portant le promoteur 7.5 Kd qui a été introduit dans le "polylinker", pour donner le pTG1171.

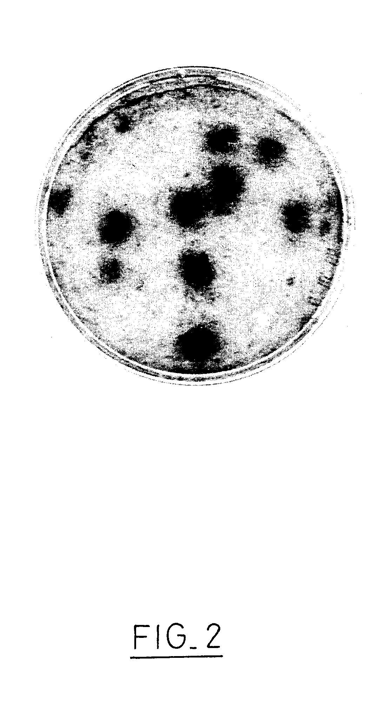

Figure 2 :    photographie d'une boîte de culture de fibroblastes de poulet montrant les plages de virus du Fowlpox recombinant exprimant la $\beta$-galactosidase et qui apparaissent colorées en bleu en présence de X-Gal.

Figure 3 :    Construction de deux vecteurs permettant le transfert du gène codant pour la protéine F du virus de la rougeole dans le génome du virus du Fowlpox.

Les séquences d'ADN du génome du Fowlpox sont représentées par des arcs de cercle au trait double annotés selon les cadres de lecture qu'elles renferment . soit $F_6$-$F_7$, soit $F_9$. L'arc de cercle au trait unique représente le vecteur bactérien pPolyII. Le promoteur P7,5 est schématisé par une flèche épaisse indiquant la direction de la transcription. Les gènes codant pour la $\beta$-galactosidase d'E. coli et la protéine F du virus de la rougeole sont annotés lac Z et F measles respectivement et leur direction de lecture normale est la même que celle du promoteur P7 5 situé en aval de chaque gène. Les sites désignés B ° correspondent à l'endroit d'une ligation entre un site BglII et un site BamHI.

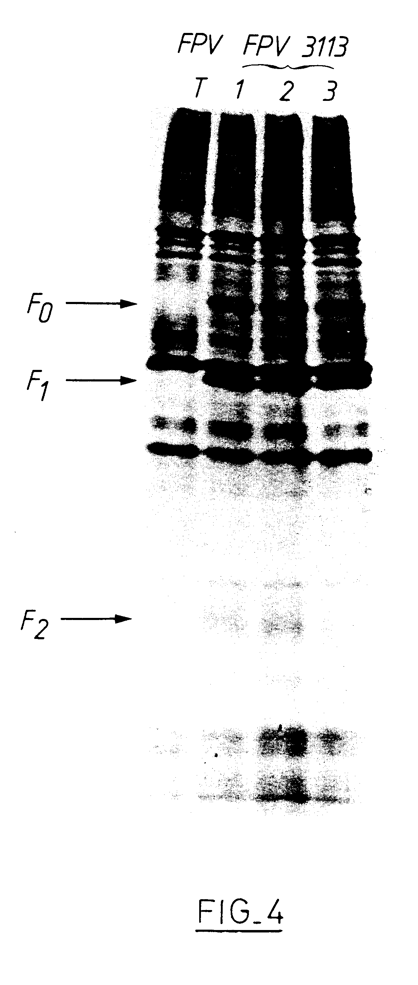

Figure 4 :    Mise en évidence de la synthèse de la protéine F du virus de la rougeole dans des cellules infectées par le virus FPV3113.

Les protéines de cellules de poulet infectées par la souche sauvage du Fowlpox (piste annotée T) ou trois isolats de la souche recombinante FPV3113 (pistes annotées 1,2 et 3) ont été immunoprécipitées avec un sérum de cobaye antirougeole puis analysées par électrophorèse sur gel de polyacrylamide. L'autoradiographie du gel séché est présentée. Les différentes formes de la protéine F ($F_0$, $F_1$, $F_2$) sont désignées par des flèches.

Exemple 1 Sélection et caractérisation de mutants thermosensibles (ts) du virus du Fowlpox.

On utilise une souche vaccinale du virus de la variole des poules, ou Fowlpox, par exemple le vaccin produit par les laboratoires Salsbury (Poxine[R], Laboratoire Salsbury, 56 Rue Delprier, 37000 TOURS, France).

La multiplication du virus est mesurée à 3 températures :

```
                                 titre après 6 jours :
          33°C                        4,1.10^5 ufp/ml
          37°C                        4,1.10^5 ufp/ml
          39,5°C                      4,2.10^5 ufp/ml
```

Le virus non muté se multiplie aussi efficacement aux 3 températures.

Un stock de virus cloné et amplifié à 39,5°C est traité à la nitrosoguanidine à 10 ou 20 µg/ml. Après ce traitement mutagène, le virus est étalé, à une dilution appropriée pour obtenir des plages bien isolées, sur cultures de fibroblastes d'embryons de poulets. Les cultures sont incubées à 33°C (température permissive) pendant 6 ou 7 jours puis colorées au rouge neutre pour faire apparaître les plages de virus.

Les cultures sont ensuite incubées à 39,5°C (température restrictive) pendant 2 jours. Les plages dont la taille n'a pas augmenté sont considérées comme thermosensibles. Les virus de quelques plages sont prélevés et amplifiés à 33°C puis retirés à 33 et 39,5°C pour confirmer leur caractère ts. Le tableau suivant présente les titres obtenus aux 2 températures.

| Titrage à : | 33°C | 39,5°C | |
|---|---|---|---|
| Virus Fowlpox témoin | $1.10^5$ | $1.10^5$ | ufp/ml |
| mutant ts1 | $2.10^5$ | <10 | ufp/ml |
| mutant ts11 | $2.10^5$ | <10 | ufp/ml |
| mutant ts24 | $2.10^5$ | <10 | ufp/ml |

Exemple 2 Mise au point des conditions de recombinaison entre virus mutés ts et ADN purifié de virus non muté.

Dans les conditions restrictives, les mutants ts ne se multiplient plus.

L'ADN purifié des virus de la famille des poxviridae n'est pas infectieux.

Si l'introduction dans une même cellule d'un virus ts et d'un ADN purifié donne lieu à une multiplication de virus, ceux-ci résultent d'une complémentation ou d'une recombinaison in vivo entre les 2 génomes.

On infecte des tapis de fibroblastes d'embryons de poulets avec les virus ts ($4.10^4$ ufp/$10^6$ cellules). Après 1 heure d'adsorption, on renouvelle les cultures en milieu frais et on les incube pendant 2 heures à température permissive (33-37°C).

Ensuite, on élimine le milieu et on adsorbe l'ADN purifié du virus non muté, sous forme de précipité de phosphate de calcium (1 µg d'ADN dans 5 µl de tampon, pour 4 boîtes de cultures).

Après 1 heure d'adsorption, on renouvelle en milieu frais et on incube pendant 2 heures à température non permissive (39,5°) puis on effectue un choc au glycérol (10%).

Ensuite, les cultures sont remises à incuber pendant 5 jours à 39,5°C.

Les plages de virus sont comptées, pour la moitié, directement sur les boîtes où la manipulation a été effectuée, et pour la moitié, après amplification (c'est-à-dire après congélation des premières boîtes suivie d'un deuxième cycle de multiplication).

| | Nombre de plages de virus | | | |
|---|---|---|---|---|
| | sans amplification | | après amplification | |
| | sans ADN | + ADN | sans ADN | + ADN |
| ts1 | 0 | 15 | 10 | $3.10^4$ |
| ts11 | 0 | 2 | 10 | $1.10^4$ |
| ts24 | 0 | 3 | 10 | $1.10^4$ |

L'expérience montre que l'ADN purifié du virus du Fowlpox sauvage peut être réactivé par les mutants ts pour donner une progénie infectieuse.

Le mutant ts1 a été retenu pour les expériences suivantes.

Exemple 3 Construction d'un vecteur destiné au transfert d'un ADN hétérologue dans le virus du Fowlpox.

Pour transférer une séquence d'ADN étranger dans le génome d'un virus, cette séquence doit être intégrée au milieu de séquences intactes du virus de façon à permettre une double recombinaison homologue.

L'ADN étranger doit être inséré dans une zone non essentielle du virus de manière à ne pas empêcher sa multiplication.

Un vecteur a été construit pour favoriser l'insertion d'un ADN étranger dans une zone intergénique non codante du virus du Fowlpox.

a) Insertion d'un fragment d'ADN du Fowlpox dans un plasmide bactérien : construction du pTG1170.

Un fragment d'ADN du Fowlpox présentant une importante homologie avec l'ADN du virus de la vaccine a été décrit par Drillien et al. (1987).

Ce fragment (homologue au fragment HindIII J du virus de la vaccine) porte 5 séquences ouvertes de lecture (ORF) et se distingue de la vaccine par l'absence du gène F8 qui est remplacé par une zone intergénique non codante de 32 paires de bases.

Cette zone intergénique a été choisie comme site d'insertion de l'ADN étranger.

Un large fragment d'ADN qui entoure cette zone a été récupéré pour servir de séquence homologue permettant la recombinaison et l'intégration dans le génome viral du Fowlpox. Ce fragment a été obtenu après digestion par EcoRI et PstI. Le site EcoRI est situé en amont de l'ORF6 et le site PstI est situé dans l'ORF9 (voir schéma ci-dessus). Le fragment EcoRI-PstI a été intégré dans un vecteur M13TG131 (Kieny et al. 1983), pour donner le M13TG188.

L'introduction dans M13 permet d'effectuer une mutagénèse localisée, afin d'introduire des sites de reconnaissance pour des enzymes de restriction qui permettront l'insertion d'ADN étranger.

On a créé 3 sites : XhoI, BamHI et EcoRI, dans la région intergénique de 32 pb, à l'aide de l'oligonucléotide de synthèse suivant :

5' TTAAAAAGGAATTGACTCGAGGGATCCGAATTCAAGAAAATATTTAT 3'

La construction portant l'insert synthétique est appelée M13TG195. La présence des sites de restriction a été vérifiée avec les enzymes correspondants.

La séquence d'ADN du Fowlpox mutée a été récupérée à partir du M13TG195 sous forme de fragment BglII-PstI (le site BglII est situé au début de l'ORF6, Drillien et al. 1987) et introduite dans un vecteur de clonage dérivé du pML2 portant un adaptateur synthétique comportant plusieurs sites de restriction, le POLYII décrit par Lathe et al. (1987).

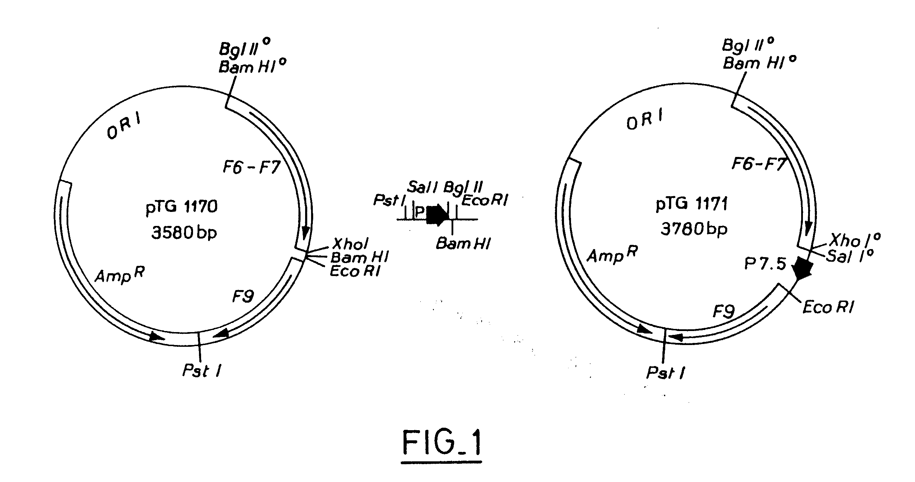

L'insertion du fragment BglII-PstI du Fowlpox dans le vecteur POLYII ouvert par BamHI et PstI donne le plasmide pTG1170, qui est schématisé dans la figure 1.

b) Insertion d'un promoteur de poxvirus dans le vecteur pTG1170.

Pour obtenir l'expression d'un gène étranger par un poxvirus, il est nécessaire de placer ce gène sous le contrôle d'un promoteur de poxvirus.

Dans un premier temps, on a utilisé un promoteur bien caractérisé du virus de la vaccine, le promoteur du gène de la protéine 7.5 K, appelé en abrégé "promoteur 7.5 K". On a introduit, dans le vecteur pTG1170, le promoteur 7.5 K du virus de la vaccine, récupéré sous forme de fragment SalI-EcoRI à partir d'un vecteur M13TG7.5K décrit par Kieny et al. (1984).

Cette séquence promoteur a été introduite entre les sites XhoI et EcoRI de l'adaptateur synthétique du pTG1170 (voir figure 1).

La construction résultante, pTG1171 porte donc le promoteur 7.5 K inséré entre les ORF6-7 et l'ORF9 du Fowlpox, avec un site BamHI (apporté par la séquence P7.5K) et EcoRI immédiatement en aval.

Exemple 4 Insertion d'un gène étranger dans le vecteur pTG1171.

Un gène modèle a été utilisé pour démontrer l'utilité du vecteur et du système de sélection de recombinants du virus du Fowlpox : le gène de la $\beta$-galactosidase de E. coli. Ce gène a été choisi parce que son expression efficace peut aisément être détectée par l'addition de X-Gal (5-bromo-4-chloro-3-indolyl-$\beta$ D galactopyranoside) qui engendre une coloration bleue sous l'action de la $\beta$-galactosidase.

Le gène lacZ codant pour la $\beta$-galactosidase de E. coli peut être récupéré, par exemple, à partir du plasmide pCH110 (Hall et al. 1983). Le gène lacZ a été excisé par BamHI et HindIII et inséré dans le vecteur de clonage POLYII (cité plus haut) puis repris sous forme de fragment BglII-BamHI pour être inséré dans le pTG1171 ouvert par BamHI et traité à la phosphatase.

La construction portant le gène lacZ en aval du promoteur 7.5 K est appelé pTG1177.

Exemple 5 Expression d'un gène étranger par un virus recombinant du Fowlpox.

L'ADN du plasmide pTG1177 (100 ng/$10^6$ cellules) a été transfecté dans des cellules d'embryons de poulet préalablement infectées par le virus Fowlpox ts1, selon le protocole décrit dans l'exemple 2.

Après 5 jours d'incubation à 39,5°C, on récolte les virus recombinants après avoir lysé les cellules par congélation.

Les virus sont titrés sur cultures de fibroblastes d'embryons de poulet, sous milieu gélosé. Après 5 jours, les plages du virus sont révélées au X-Gal (solution à 30 mg/ml diluée à 1/100 en milieu PBS + agarose 1%).

Les plages de virus se colorent en bleu, ce qui indique la présence de $\beta$-galactosidase fonctionnelle.

Exemple 6 Construction d'un deuxième vecteur destiné au transfert d'ADN hétérologue dans le virus du Fowlpox.

a) Un autre vecteur permettant le transfert d'ADN hétérologue dans le virus Fowlpox a été construit en conservant une région intergénique plus importante entre les ORF F7 et F9. Pour réaliser cet objectif, une première mutagénèse localisée a été effectuée sur le phage M13TG188 (décrit dans l'exemple 3) avec l'oligonucléotide de synthèse suivant

5′ AACAACCTAATATCACTATAGGATCCTTGTTAAAAAGGAAT 3′Ceci donne le phage M13TG2123 et a pour résultat d'introduire un site BamHI dans la partie 3′ codante de l'ORF F7. Une deuxième mutagénèse a ensuite été effectuée sur M13TG2123 avec l'oligonucléotide suivant 5′ CCTAATATCACTACTGAAGCGCAACTAGGATCCTTGTTAA 3′

Dans le phage résultant, M13TG2125, la distance intergénique entre l'ORF F7 et l'ORF F9 est augmentée d'une quinzaine de nucléotides et les codons situés en 3′ de l'ORF F7 sont remplacés par des codons équivalents, codant pour les mêmes acides aminés.

La séquence d'ADN du Fowlpox muté a été récupérée à partir du M13TG2125 sous forme de fragment BglII-PstI et introduit dans le vecteur POLYII comme dans l'exemple 3 pour générer le plasmide pTG2134.

b) Insertion d'un promoteur de poxvirus dans le vecteur pTG2134.

Le promoteur du gène de la protéine 7,5 K du virus de la vaccine récupéré sous forme de fragment BglII-BamHI d'un vecteur M13TG2119 (dérivé du M13TG7.5K décrit dans l'exemple 3) a été inséré dans le site BamHI du plasmide pTG2134. Ceci a donné naissance à deux vecteurs pTG2135 et pTG2136 qui se distinguent entre eux par l'orientation du promoteur. Dans le plasmide pTG2135, le promoteur est orienté dans le même sens que les ORF F7 et F9 tandis que dans le plasmide pTG2136, l'orientation est inversée.

**Exemple 7** Insertion d'un gène étranger dans les vecteurs pTG2135 et pTG2136.

Comme dans l'exemple 4, le gène de la $\beta$-galactosidase, sous forme d'un fragment BglII-BamHI, a été inséré dans les vecteurs pTG2135 et pTG2136 préalablement ouverts par BamHI et traités par la phosphatase. Ces manipulations ont donné naissance au vecteur pTG2137 où le gène de la $\beta$-galactosidase est orienté dans le même sens que les gènes des ORFs F7 et F9 et au vecteur pTG2138 dans lequel l'orientation du gène de la $\beta$-galactosidase est inversée.

L'ADN du pTG2137 a été transfecté dans des cellules d'embryons de poulet infectées par le virus du Fowlpox tsl, comme dans l'exemple 5.

Des plages de virus recombinants sont observées après 5 jours et elles se colorent en bleu après addition de X-Gal.

**Exemple 8** Insertion d'un gène étranger distinct du gène de la $\beta$-galactosidase dans le génome du Fowlpox et expression dans des cellules infectées par des virus recombinants du Fowlpox.

### A. Construction des virus recombinants

La construction d'un vecteur (pTG2137) qui permet de transférer dans le génome du Fowlpox le gène de la $\beta$-galactosidase et de reconnaître les virus recombinants grâce à la couleur bleue des plages du Fowlpox en présence d'un substrat chromogène (X-gal) a été décrite dans le brevet principal. On peut de plus sélectionner pour l'intégration d'un deuxième gène dans le génome du Fowlpox sans que celui-ci confère au virus un nouveau phénotype facilement détectable. Ceci est illustré pour le cas du gène codant pour la protéine F du virus de la rougeole.

Les étapes sont les suivantes :

Dans un premier temps, un deuxième promoteur P7,5 a été ajouté au plasmide pTG2137 afin d'assurer la transcription d'un deuxième gène indépendant de la $\beta$-galactosidase. Le promoteur P7,5 a été obtenu sous forme d'un fragment BglII-BamHI excisé du phage M13TG2119 décrit auparavant. Le promoteur P7,5 a été intégré en aval du gène $\beta$-gal dans le site BamHI du vecteur pTG2137 soit dans la même orientation que le premier promoteur P7,5 pour donner le plasmide pTG2149, soit dans l'orientation opposée pour donner le plasmide pTG2150.

Ensuite, le cADN codant pour le gène F du virus de la rougeole a été inséré en aval du nouveau promoteur P7,5 dans les deux vecteurs pTG2149 et pTG2150. D'abord, le cADN codant pour la partie N-terminale de la protéine F a été excisé par coupure de pTG1172 avec les enzymes BglII et BamHI et le fragment obtenu a été inséré dans le site BamHI de pTG2149 ou pTG2150 de façon à respecter le sens de la transcription correcte du gène F par rapport au promoteur P7,5 et donner naissance respectivement aux plasmides pTG2192 et pTG2193.

Le cADN codant pour la partie C terminale du gène F a ensuite été isolé de pTG1173 par coupure avec l'enzyme BamHI et le fragment ajouté au pTG2192 préalablement coupé par le même enzyme de façon à reconstituer un gène F intact. La partie C-terminale du gène F dans le plasmide pTG1173 contient une région poly A qui avait été délétée dans le phage M13TG2143. Ce dernier a fourni la partie C-terminale du gène F sous forme d'un fragment BglII-BamHI ajouté au plasmide pTG2193 coupé par BamHI. Le plasmide formé lors de la ligation précédente est appelé pTG3113.

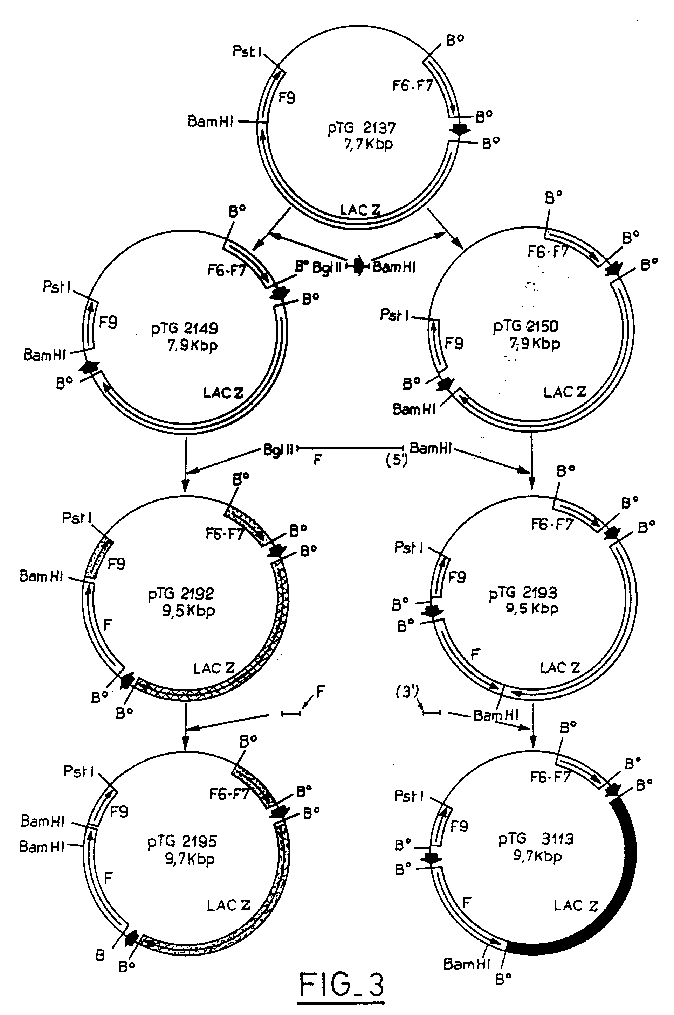

Donc, les plasmides pTG2195 et pTG3113 contiennent chacun le gène F complet du virus de la rougeole sous le contrôle du promoteur P7,5 soit dans la même orientation que le premier promoteur P7,5 qui gouverne l'expression du gène de la $\beta$-galactosidase (pTG2195), soit dans l'orientation opposée. L'ensemble des clonages réalisés qui ont généré des plasmides recombinants est résumé dans la figure 3.

Les plasmides recombinants pTG2195 et pTG3113 ont servi à transférer le gène de la $\beta$-galactosidase et le gène de la protéine F dans le génome du virus du Fowlpox selon les techniques décrites dans les exemples 2 et 5.

Des plages de virus recombinant du Fowlpox dénommées FPV2195 et FPV3113 ont été repérées grâce à leur couleur bleue en présence de X-gal. Le virus contenu dans les plages a été repris, amplifié par infection de cellules d'embryons de poulet, puis réétalé à nouveau. Des plages bleues ont été repérées et la procédure précédente répétée.

Cette technique de clonage successif a montré que le virus FPV3113, après avoir été débarrassé du virus non recombinant qui le contaminait initialement, présente un phénotype β-galactosidase stable. Par contre, le virus FPV2195 soumis aux mêmes cycles de clonage successif se révèle instable, à savoir qu'une plage bleue isolée donne environ 2% de plages incolores, incapables de coder pour la β-galactosidase. Le virus FPV2195 isolé au départ a donc tendance à ségréger le gène β-gal tout en conservant le gène qui code pour la protéine F ; le virus qui en résulte est appelé FPV2195 β-gal⁻. La perte du gène β-gal a lieu vraisemblablement à la suite d'évènements de recombinaison inter- ou intramoléculaires entre les deux promoteurs P7,5 placés en répétition directe par rapport au gène β-gal. Cette méthode a l'avantage de permettre la sélection initiale pour l'intégration d'un gène choisi (ici le gène F) grâce au marqueur β-gal qui y est associé physiquement puis l'élimination du marqueur par un mécanisme de recombinaison naturelle du virus et enfin la réutilisation éventuelle du marqueur pour l'insertion d'un autre gène ou du même dans une autre région du génome.

B. Expression de la protéine F dans des cellules infectées par FPV2195 β-gal⁻ et FPV3113

Des cellules d'embryons de poulet ont été infectées avec environ 1 ufp par cellule de FPV3113, FPV2195 β-gal⁻ ou FPV de type sauvage. Après 40 heures d'infection, les cellules ont été marquées à la méthionine $^{35}$S pendant 4 heures, puis lysées avec un tampon d'immunoprécipitation. La protéine F contenue dans les lysats de cellules infectées avec les recombinants a été immunoprécipitée avec un sérum polyclonal de cobaye dirigé contre l'ensemble des protéines du virus de la rougeole en présence de la protéine A de Staphylocoque aureus fixée au sepharose. Les protéines immunoprécipitées ont été analysées sur gel de polyacrylamide en présence de SDS ; le gel séché a été soumis à autoradiographie. Les résultats présentés (figure 4) pour le recombinant FPV3113 démontrent la présence du produit primaire de traduction de l'ARNm correspondant au gène F (F°) ainsi que les produits de clivage $F_1$ et $F_2$.

REFERENCES

Brown, T.D.K. Span 28, 68-70 (1985).

Chakrabarti, S., Robert-Guroff, M., Wong-Staal, F., Gallo, R.C. and Moss, B. Nature 320, 535-540 (1986).

Drillien, R., Spehner, D., Villeval, D. and Lecocq, J.P. Virology 160, in press (1987).

Hall, C.V., Jacob, P.E., Ringold, G.M. and Lee, F. J. Mol. Appl. Gen. 2, 101-109 (1983).

Hu, S.L., Kosowski, S.G. and Dabrymple, J.M. Nature 320, 537-540 (1986).

Kieny, M.P., Lathe, R. and Lecocq, J.P. Gene 26, 91-99 (1983).

Kieny, M.P., Lathe, R., Drillien, R., Spehner, D., Skory, S., Schmitt, D., Wiktor, T., Koprowski, H. and Lecocq, J.P. Nature 312, 163-166 (1984).

Kieny, M.P., Rautmann, G., Schmitt, D., Dott, K., Wain-Hobson, S., Alizon, M., Girard, M., Chamaret, S., Laurent, A., Montagnier, L. and Lecocq, J.P. Biotechnology 4, 790-795 (1986).

Lathe, R., Vilotte, J.L. and Clark, A.J. Gene in press (1987).

Mackett, M., Smith, G.L. and Moss, B. PNAS U.S.A. 79, 7415-7419 (1982).

Moss, B. In "Virology" (Ed. B.N. Fields) pp. 685-703 Raven Press. New York (1985).

Panicali, D., Davis, S.W., Weinberg, R.L., Paoletti, E. Proc. Natl. Acad. Sci. USA 80, 5364-5368 (1983).

Sam, C.K. and Dumbell, K.R. Ann. Virol. (Inst. Pasteur) 132, 135-150 (1981).

Smith, G.L., Mackett, M., Moss, V. Nature 302, 490-495 (1983).

Taylor, J., Weinberg, R., Desmettre, P., Paoletti, E., In : Modern approaches to new vaccines including prevention of AIDS. Cold Spring Harbor. New York. Abstracts of the 1987 meeting (9-13 September)pp.120.

**Revendications**

**Revendications pour les Etats contractants suivants : AT, BE, CH, DE, FR, GB, IT, LI, LU, NL, SE**

**1.** Virus du Fowlpox recombinant, caractérisé en ce qu'il dérive d'une souche atténuée de virus du Fowlpox et comporte, dans une zone intergénique non codante et non essentielle du virus du Fowlpox une séquence d'ADN codant pour tout ou partie d'une protéine hétérologue au virus du Fowlpox ainsi que les éléments susceptibles d'assurer l'expression de cette protéine dans une cellule infectée par ledit virus recombinant.

**2.** Virus du Fowlpox selon la revendication 1, caractérisé en ce que la zone intergénique non codante est la zone située entre les ORF 7 et 9 du fragment HindIII de 1.3 K paires de bases de l'ADN viral.

**3.** Virus du Fowlpox selon la revendication 2, caractérisé en ce que la séquence d'ADN codant pour tout ou partie de la protéine hétérologue est insérée entre le codon de fin de traduction de l'ORF 7 et la région intergénique de 32 paires de bases située immédiatement en amont de l'ORF 9.

**4.** Virus du Fowlpox selon l'une des revendications 1 à 3, caractérisé en ce que ledit virus recombinant dérive d'une souche vaccinale du virus du Fowlpox.

**5.** Virus du Fowlpox selon la revendication 4, caractérisé en ce que ledit virus recombinant dérive d'une souche de virus du Fowlpox thermosensible.

**6.** Virus du Fowlpox selon l'une des revendications 1 à 5, caractérisé en ce qu'il comporte un gène codant pour un marqueur de sélection qui s'exprime dans une cellule infectée par ledit virus recombinant.

**7.** Virus du Fowlpox selon l'une des revendications 1 à 6, caractérisé en ce que la séquence d'ADN code pour une protéine induisant une réponse immunitaire contre un virus hétérologue.

**8.** Virus du Fowlpox selon la revendication 7, caractérisé en ce que ledit virus hétérologue est choisi parmi le virus de la maladie de Newcastle, le virus de la bronchite infectieuse et le virus de la maladie de Marek.

**9.** Virus du Fowlpox selon l'une des revendications 1 à 7, caractérisé en ce que la séquence d'ADN code pour une protéine induisant une réponse immunitaire contre un Morbillivirus.

**10.** Virus du Fowlpox selon la revendication 9, caractérisé en ce que ledit Morbillivirus est choisi parmi le virus de la rougeole, le virus de la maladie de Carré, le virus de la peste bovine et le virus de la peste des petits ruminants.

**11.** Virus du Fowlpox selon la revendication 10, caractérisé en ce que la séquence d'ADN code pout tout ou partie d'au moins une protéine de structure du virus de la rougeole.

**12.** Virus du Fowlpox selon l'une des revendications 1 à 7, caractérisé en ce que la séquence d'ADN code pour un facteur intervenant dans le métabolisme de la volaille.

**13.** Procédé d'obtention d'un virus recombinant selon l'une des revendications 1 à 12, caractérisé en ce qu'on transfecte une culture de cellules compétentes avec l'ADN d'un plasmide qui comporte au moins un gène hétérologue au virus du Fowlpox, sous le contrôle d'un promoteur de poxvirus, en particulier le promoteur de la protéine 7,5 K du virus de la vaccine, flanqué de part et d'autre de l'extrémité 3' de l'ORF 7 et de l'extémité 5' de l'ORF 9,
ladite culture ayant été préalablement infectée par un virus du Fowlpox.

**14.** Culture de cellules eucaryotes infectée par un virus du Fowlpox recombinant selon l'une des revendications 1 à 12.

**15.** Culture de cellules selon la revendication 14, caractérisée en ce qu'il s'agit de cellules aviaires.

**16.** Vaccin caractérisé en ce qu'il contient un virus du Fowlpox selon l'une des revendications 1 à 12.

**17.** Vaccin caractérisé en ce qu'il contient un virus du Fowlpox obtenu par réplication dans les cultures de cellules selon l'une des revendications 14 ou 15, caractérisé en ce que le virus du Fowlpox est vivant.

**18.** Antisérum contenant les anticorps dressés contre le virus du Fowlpox selon l'une des revendications 1 à 12.

EP 0 314 569 B1

**19.** Procédé de préparation d'une protéine d'intérêt industriel ou thérapeutique, caractérisé en ce qu'on cultive une cellule eucaryote selon les revendications 14 ou 15 et qu'on récupère la protéine obtenue.

**Revendications pour les Etats contractants suivants : ES, GR**

**1.** Procédé d'obtention d'un virus du Fowlpox recombinant, caractérisé en ce qu'on transfecte une culture de cellules compétentes, préalablement infectée par une souche atténuée de virus du Fowlpox, avec un ADN qui comporte une séquence codant pour tout ou partie d'une protéine hétérologue au virus du Fowlpox ainsi que les éléments susceptibles d'assurer l'expression de cette protéine dans une cellule infectée par ledit virus recombinant, et en ce que cet ADN est inséré dans une zone intergénique non codante et non essentielle du virus du Fowlpox.

**2.** Procédé d'obtention d'un virus du Fowlpox selon la revendication 1, caractérisé en ce que la zone intergénique non codante est la zone située entre les ORF 7 et 9 du fragment HindIII de 1.3 K paires de bases de l'ADN viral.

**3.** Procédé d'obtention d'un virus du Fowlpox selon la revendication 2, caractérisé en ce que la séquence d'ADN codant pour tout ou partie de la protéine hétérologue est insérée entre le codon de fin de traduction de l'ORF 7 et la région intergénique de 32 paires de bases située immédiatement en amont de l'ORF 9.

**4.** Procédé d'obtention d'un virus du Fowlpox selon l'une des revendications 1 à 3, caractérisé en ce que ledit virus recombinant dérive d'une souche vaccinale du virus du Fowlpox.

**5.** Procédé d'obtention d'un virus du Fowlpox selon la revendication 4, caractérisé en ce que ledit virus recombinant dérive d'une souche de virus du Fowlpox thermosensible.

**6.** Procédé d'obtention d'un virus du Fowlpox selon l'une des revendications 1 à 5, caractérisé en ce que le virus comporte un gène codant pour un marqueur de sélection qui s'exprime dans une cellule infectée par ledit virus recombinant.

**7.** Procédé d'obtention d'un virus du Fowlpox selon l'une des revendications 1 à 6, caractérisé en ce que la séquence d'ADN code pour une protéine induisant une réponse immunitaire contre un virus hétérologue.

**8.** Procédé d'obtention d'un virus du Fowlpox selon la revendication 7, caractérisé en ce que ledit virus hétérologue est choisi parmi le virus de la maladie de Newcastle, le virus de la bronchite infectieuse et le virus de la maladie de Marek.

**9.** Procédé d'obtention d'un virus du Fowlpox selon l'une des revendications 1 à 7, caractérisé en ce que la séquence d'ADN code pour une protéine induisant une réponse immunitaire contre un Morbillivirus.

**10.** Procédé d'obtention d'un virus du Fowlpox selon la revendication 9, caractérisé en ce que ledit Morbillivirus est choisi parmi le virus de la rougeole, le virus de la maladie de Carré, le virus de la peste bovine et le virus de la peste des petits ruminants.

**11.** Procédé d'obtention d'un virus du Fowlpox selon la revendication 10, caractérisé en ce que la Séquence d'ADN code pour tout ou partie d'au moins une protéine de structure du virus de la rougeole.

**12.** Procédé d'obtention du virus du Fowlpox selon l'une des revendications 1 à 7, caractérisé en ce que la séquence d'ADN code pour un facteur intervenant dans le métabolisme de la volaille.

**13.** Procédé d'obtention d'un virus recombinant selon l'une des revendications 1 à 12, caractérisé en ce qu'on transfecte une culture de cellules compétentes avec l'ADN d'un plasmide qui comporte au moins un gène hétérologue au virus du Fowlpox, sous le contrôle d'un promoteur de poxvirus, en particulier le promoteur de la protéine 7,5 K du virus de la vaccine, flanqué de part et d'autre de l'extrémité 3' de l'ORF 7 et de l'extrémité 5, de l'ORF 9,

EP 0 314 569 B1

ladite culture ayant été préalablement infectée par un virus du Fowlpox.

**14.** Procédé d'obtention d'une culture de cellules eucaryotes infectée par un virus du Fowlpox recombinant, caractérisé en ce qu'on récupère la culture obtenue par le procédé selon l'une des revendications 1 à 13.

**15.** Procédé d'obtention d'une culture de cellules selon la revendication 14, caractérisé en ce qu'il s'agit de cellules aviaires.

**16.** Procédé de préparation d'un vaccin, caractérisé en ce qu'on mélange un virus du Fowlpox obtenu par le procédé selon l'une des revendications 1 à 13, avec un support pharmaceutiquement acceptable.

**17.** Procédé d'obtention d'un vaccin selon la revendication 16, caractérisé en ce qu'il contient un virus du Fowlpox obtenu par réplication dans des cultures de cellules préparées selon l'une des revendications 14 et 15, et en ce que ledit virus du Fowlpox est vivant.

**18.** Procédé de préparation d'un antisérum, caractérisé en ce qu'on recueille des anticorps dressés contre le virus du Fowlpox préparé selon l'une des revendications 1 à 13.

**19.** Procédé de préparation d'une protéine d'intérêt industriel ou thérapeutique, caractérisé en ce qu'on cultive une cellule eucaryote selon les revendications 14 ou 15 et qu'on récupère la protéine obtenue.

**Claims**
**Claims for the following Contracting States : AT, BE, CH, DE, FR, GB, IT, LI, LU, NL, SE**

**1.** A recombinant fowlpox virus, which is derived from an attenuated strain of fowlpox virus and contains, in a non-essential and non-coding intergenic region of the fowlpox virus, a DNA sequence coding for all or part of a protein which is heterologous to the fowlpox virus, as well as the elements capable of providing for the expression of this protein in a cell infected with the said recombinant virus.

**2.** The fowlpox virus as claimed in claim 1, in which the non-coding intergenic region is the region situated between ORFs 7 and 9 of the 1.3-kbp HindIII fragment of the viral DNA.

**3.** The fowlpox virus as claimed in claim 2, in which the DNA sequence coding for all or part of the heterologous protein is inserted between the end of translation codon of the ORF 7 and the intergenic region of 32 base pairs located immediately upstream the ORF 9.

**4.** The fowlpox virus as claimed in one of claims 1 to 3, in which the said recombinant virus is derived from a vaccinal strain of the fowlpox virus.

**5.** The fowlpox virus as claimed in claim 4, in which the said recombinant virus is derived from a temperature-sensitive strain of fowlpox virus.

**6.** The fowlpox virus as claimed in one of claims 1 to 5, which contains a gene coding for a selection marker which is expressed in a cell infected with the said recombinant virus.

**7.** The fowlpox virus as claimed in one of claims 1 to 6, in which the DNA sequence codes for a protein that induces an immune response against a heterologous virus.

**8.** The fowlpox virus as claimed in claim 7, in which the said heterologous virus is chosen from Newcastle disease virus, infectious bronchitis virus and Marek's desease virus.

**9.** The fowlpox virus as claimed in one of claims 1 to 7, in which the DNA sequence codes for a protein that induces an immune response against a Morbillivirus.

**10.** The fowlpox virus as claimed in claim 9, in which the said Morbillivirus is chosen from measles virus, Carre's disease virus, rinderpest virus and the virus of little ruminant's pest (pest des petits ruminants).

12

**11.** The fowlpox virus as claimed in claim 10, in which the DNA sequence codes for all or part of at least one structural protein of the measles virus.

**12.** The fowlpox virus as claimed in one of claims 1 to 7, in which the DNA sequence codes for a factor participating in the metabolism of poultry.

**13.** A process for obtaining a recombinant virus as claimed in one of claims 1 to 12, wherein a culture of competent cells is transfected with the DNA of a plasmid containing at least one gene which is heterologous to the fowlpox virus, under the control of a poxvirus promoter, in particular the 7.5 K protein promoter of the vaccinia virus flanked on each side by the 3' end of ORE 7 and the 5' end of ORE 9, the said culture having been infected beforehand with a fowlpox virus.

**14.** A eukaryotic cell culture infected with a recombinant fowlpox virus as claimed in one of claims 1 to 12.

**15.** The cell culture as claimed in claim 14, in which the cells are avian cells.

**16.** A vaccine, which contains a fowlpox virus as claimed in one of claims 1 to 12.

**17.** A vaccine, which contains a fowlpox virus obtained by replication in the cell cultures as claimed in one of claims 14 and 15, in which the fowlpox virus is live.

**18.** An antiserum containing antibodies raised against the fowlpox virus as claimed in one of claims 1 to 12.

**19.** A process for preparing a protein of industrial or the therapeutic importance, wherein a eukaryotic cell is cultured as claimed in claim 14 or 15, and wherein the protein obtained is recovered.

**Claims for the following Contracting States : ES, GR**

**1.** Process for obtaining a recombinant fowlpox virus wherein a culture of competent cells, which has been beforehand infected with an attenuated strain of fowlpox virus, is transfected with a DNA containing a sequence coding for all or part of a protein which is heterologous to the fowlpox virus, as well as the elements capable of providing for the expression of this protein in a cell infected with the said recombinant virus, and wherein said DNA is inserted in a non-essential and non-coding intergenic region of the fowlpox virus.

**2.** Process for obtaining a fowlpox virus as claimed in claim 1, in which the non-coding intergenic region is the region situated between ORFs 7 and 9 of the 1.3-kbp HindIII fragment of the viral DNA.

**3.** Process for obtaining a fowlpox virus as claimed in claim 2, in which the DNA sequence coding for all or part of the heterologous protein is inserted between the end of translation codon of the ORF 7 and the intergenic region of 32 base pairs located immediately upstream the ORF 9.

**4.** Process for obtaining a fowlpox virus as claimed in one of claims 1 to 3, in which the said recombinant virus is derived from a vaccinal strain of the fowlpox virus.

**5.** Process for obtaining a fowlpox virus as claimed in claim 4, in which the said recombinant virus is derived from a temperature-sensitive strain of fowlpox virus.

**6.** Process for obtaining a fowlpox virus as claimed in one of claims 1 to 5, in which said virus contains a gene coding for a selection marker which is expressed in a cell infected with the said recombinant virus.

**7.** Process for obtaining a fowlpox virus as claimed in one of claims 1 to 6, in which the DNA sequence codes for a protein that induces an immune response against a heterologous virus.

**8.** Process for obtaining a fowlpox virus as claimed in claim 7, in which the said heterologous virus is chosen from Newcastle disease virus, infectious bronchitis virus and Marek's desease virus.

**9.** Process for obtaining a fowlpox virus as claimed in one of claims 1 to 7, in which the DNA sequence codes for a protein that induces an immune response against a Morbillivirus.

**10.** Process for obtaining a fowlpox virus as claimed in claim 9, in which the said Morbillivirus is chosen from measles virus, Carre's disease virus, rinderpest virus and the virus of little ruminant's pest (pest des petits ruminants).

**11.** Process for obtaining a fowlpox virus as claimed in claim 10, in which the DNA sequence codes for all or part of at least one structural protein of the measles virus.

**12.** Process for obtaining a fowlpox virus as claimed in one of claims 1 to 7, in which the DNA sequence codes for a factor participating in the metabolism of poultry.

**13.** A process for obtaining a recombinant virus as claimed in one of claims 1 to 12, wherein a culture of competent cells is transfected with the DNA of a plasmid containing at least one gene which is heterologous to the fowlpox virus, under the control of a poxvirus promoter, in particular the 7.5 K protein promoter of the vaccinia virus flanked on each side by the 3' end of ORF 7 and the 5' end of ORF 9, the said culture having been infected beforehand with a fowlpox virus.

**14.** Process for obtaining a eukaryotic cell culture infected with a recombinant fowlpox virus wherein the culture obtained by the process as claimed in one of claims 1 to 13 is recovered.

**15.** Process for obtaining a cell culture as claimed in claim 14, in which the cells are avian cells.

**16.** Process for preparing a vaccine wherein a fowlpox virus as obtained by the process as claimed in one of claims 1 to 13, is admixed with a pharmaceutically acceptable carrier.

**17.** Process for obtaining a vaccine as claimed in claim 16, wherein said vaccine contains a fowlpox virus obtained by replication in the cell cultures prepared as claimed in one of claims 14 and 15, and wherein said fowlpox virus is live.

**18.** Process for preparing an antiserum, wherein antibodies raised against the fowlpow virus prepared as claimed in one of claims 1 to 13 is recovered.

**19.** A process for preparing a protein of industrial or the therapeutic importance, wherein a eukaryotic cell is cultured as claimed in claim 14 or 15, and wherein the protein obtained is recovered.

**Patentansprüche**
**Patentsprüche für folgende Vertagsstaaten : AT, BE, CH, DE, FR, GB, IT, LI, LU, NL, SE**

**1.** Rekombinantes Geflügelpocken-Virus, dadurch gekennzeichnet, daß es aus einem abgeschwächten Stamm des Geflügelpocken-Virus stammt und in einer nicht-codierenden und nicht-essentiellen Intergen-Zone des Geflügelpocken-Virus eine DNA-Sequenz aufweist, die codiert für das gesamte oder einen Teil eines heterologen Proteins im Geflügelpocken-Virus sowie die Elemente, welche die Expression dieses Proteins in eine durch das genannte rekombinante Virus infizierte Zelle gewährleisten können.

**2.** Geflügelpocken-Virus nach Anspruch 1, dadurch gekennzeichnet, daß die nicht-codirende Intergen-Zone die Zone ist, die zwischen den ORF 7 und 9 des HindIII-Fragments mit 1,3 K Basenpaaren der Virus-DNA angeordnet ist.

**3.** Geflügelpocken-Virus nach Anspruch 2, dadurch gekennzeichnet, daß die DNA-Sequenz, die für das gesamte oder einen Teil des heterologen Proteins codiert, zwischen den Translations-Schlußcodon von ORF 7 und den Intergen-Bereich mit 32 Basenpaaren, der unmittelbar stromaufwärts von ORF 9 angeordnet ist, inseriert ist.

**4.** Geflügelpocken-Virus nach einem der Ansprüche 1 bis 3, dadurch gekennzeichnet, daß das genannte rekombinante Virus aus einem Vaccine-Stamm des Geflügelpocken-Virus stammt.

14

**5.** Geflügelpocken-Virus nach Anspruch 4, dadurch gekennzeichnet, daß das genannte rekombinante Virus aus einem wärmeempfindlichen Stamm des Geflügelpocken-Virus stammt.

**6.** Geflügelpocken-Virus nach einem der Ansprüche 1 bis 5, dadurch gekennzeichnet, daß es ein Gen aufweist, das für einen Selektions-Marker codiert, der in eine Zelle exprimiert, die durch das genannte rekombinante Virus infiziert ist.

**7.** Geflügelpocken-Virus nach einem der Ansprüche 1 bis 6, dadurch gekennzeichnet, daß die DNA-Sequenz für ein Protein codiert, das eineImmun-Antwort gegen ein heterologes Virus induziert.

**8.** Geflügelpocken-Virus nach Anspruch 7, dadurch gekennzeichnet, daß das genannte heterologe Virus ausgewählt wird unter dem Newcastle-Krankheits-Virus, dem infektiösen Bronchitis-Virus und dem Marek-Krankheits-Virus.

**9.** Geflügelpocken-Virus nach einem der Ansprüche 1 bis 7, dadurch gekennzeichnet, daß die DNA-Sequenz für ein Protein codiert, das eine Immun-Antwort gegen ein Morbilli-Virus induziert.

**10.** Geflügelpocken-Virus nach Anspruch 9, dadurch gekennzeichnet, daß das Morbilli-Virus ausgewählt wird aus dem Masern-Virus, dem Carré-Krankheits-Virus, dem Rinderpest-Virus und dem kleinen Wiederkäuerpest-Virus.

**11.** Geflügelpocken-Virus nach Anspruch 10, dadurch gekennzeichnet, daß die DNA-Sequenz für das gesamte oder mindestens einen Teil eines Proteins mit der Struktur des Masern-Virus codiert.

**12.** Geflügelpocken-Virus nach einem der Ansprüche 1 bis 7, dadurch gekennzeichnet, daß die DNA-Sequenz für einen Faktor codiert, der in den Geflügel-Stoffwechsel eingreift.

**13.** Verfahren zur Herstellung eines rekombinanten Virus nach einem der Ansprüche 1 bis 12, dadurch gekennzeichnet, daß man eine Kultur von kompetenten Zellen mit der DNA eines Plasmids transformiert, das mindestens ein heterologes Gen im Geflügelpocken-Virus aufweist, unter der Kontrolle eines Geflügelpocken-Virus-Promotors, insbesondere des Promotors des Proteins 7,5 K des Vaccine-Virus, beiderseits flankiert von dem 3'-Ende von ORF 7 und dem 5'-Ende von ORF 9, wobei die Kultur vorher mit einem Geflügelpocken-Virus infiziert worden ist.

**14.** Eukarioten-Zellenkultur, die durch ein rekombinantes Geflügelpocken-Virus nach einem der Ansprüche 1 bis 12 infiziert ist.

**15.** Zellkultur nach Anspruch 14, dadurch gekennzeichnet, daß es sich dabei um Aviär-Zellen handelt.

**16.** Vaccine (Impfstoff), dadurch gekennzeichnet, daß sie ein Geflügelpocken-Virus nach einem Ansprüche 1 bis 12 enthält.

**17.** Vaccine (Impfstoff), dadurch gekennzeichnet, daß sie ein Geflügelpocken-Virus enthält, das durch Replikation in den Zellenkulturen nach einem der Ansprüche 14 oder 15 erhalten worden ist, dadurch gekennzeichnet, daß es sich bei dem Geflügelpocken-Virus um ein lebendes Virus handelt.

**18.** Antiserum, das Antikörper enthält, die gegen das Geflügelpocken-Virus nach einem der Ansprüche 1 bis 12 gerichtet sind.

**19.** Verfahren zur Herstellung eines industriell oder therapeutisch interessanten Proteins, dadurch gekennzeichnet, daß man eine Eukarioten-Zelle nach den Ansprüchen 14 oder 15 züchtet und das erhaltene Protein gewinnt (abtrennt).

**Patentsprüche für folgende Vertragsstaaten : ES, GR**

**1.** Verfahren zur Herstellung eines rekombinanten Geflügelpocken-Virus, dadurch gekennzeichnet, daß man eine Kultur von kompetenten Zellen, die vorher mit einem geschwächten Stamm des Geflügelpokken-Virus infiziert worden ist, mit einer DNA transformiert, die eine Sequenz aufweist, die codiert für

EP 0 314 569 B1

das gesamte oder einen Teil eines heterologen Proteins im Geflügelpocken-Virus, sowie die Elemente, welche die Expression dieses Proteins in eine durch das genannte rekombinante Virus infizierte Zelle gewährleisten können, und daß diese DNA in eine nicht-codierende und nicht-essentielle Intergen-Zone des Geflügelpocken-Virus inseriert wird.

2. Verfahren zur Herstellung eines Geflügelpocken-Virus nach Anspruch 1, dadurch gekennzeichnet, daß die nicht-codierende Intergen-Zone die Zone ist, die zwischen den ORF 7 und 9 des HindIII-Fragments mit 1,3 K Basenpaaren der Virus-DNA angeordnet ist.

3. Verfahren zur Herstellung eines Geflügelpocken-Virus nach Anspruch 2, dadurch gekennzeichnet, daß die DNA-Sequenz, die für das gesamte oder einen Teil des heterologen Proteins codiert, zwischen den Translations-Schlußcodon von ORF 7 und den Intergen-Bereich von 32 Basenpaaren, der unmittelbar stromaufwärts von ORF 9 angeordnet ist, inseriert wird.

4. Verfahren zur Herstellung eines Geflügelpocken-Virus nach einem der Ansprüche 1 bis 3, dadurch gekennzeichnet, daß das genannte rekombinante Virus aus einem Vaccine-Stamm des Geflügelpocken-Virus stammt.

5. Verfahren zur Herstellung eines Geflügelpocken-Virus nach Anspruch 4, dadurch gekennzeichnet, daß das genannte rekombinante Virus aus einem wärmeempfindlichen Stamm des Geflügelpocken-Virus stammt.

6. Verfahren zur Herstellung eines Geflügelpocken-Virus nach einem der Ansprüche 1 bis 5, dadurch gekennzeichnet, daß das Virus ein Gen aufweist, das für einen Selektions-Marker codiert, der in eine mit dem genannten rekombinanten Virus infizierte Zelle exprimiert.

7. Verfahren zur Herstellung eines Geflügelpocken-Virus nach einem der Ansprüche 1 bis 6, dadurch gekennzeichnet, daß die DNA-Sequenz für ein Protein codiert, das eine Immun-Antwort gegen ein heterologes Virus induziert.

8. Verfahren zur Herstellung eines Geflügelpocken-Virus nach Anspruch 7, dadurch gekennzeichnet, daß das genannte heterologe Virus ausgewählt wird aus dem Newcastle-Krankheits-Virus, dem infektiösen Bronchitis-Virus und dem Marek-Krankheits-Virus.

9. Verfahren zur Herstellung eines Geflügelpocken-Virus nach einem der Ansprüche 1 bis 7, dadurch gekennzeichnet, daß die DNA-Sequenz für ein Protein codiert, das eine Immun-Antwort gegen ein Morbilli-Virus induziert.

10. Verfahren zur Herstellung eines Geflügelpocken-Virus nach Anspruch 9, dadurch gekennzeichnet, daß das Morbilli-Virus ausgewählt wird aus dem Masern-Virus, dem Carré-Krankheits-Virus, dem Rinderpest-Virus und dem kleinen Wiederkäuerpest-Virus.

11. Verfahren zur Herstellung eines Geflügelpocken-Virus nach Anspruch 10, dadurch gekennzeichnet, daß die DNA-Sequenz für das gesamte oder mindestens einen Teil eines Proteins mit der Struktur des Masern-Virus codiert.

12. Verfahren zur Herstellung des Geflügelpocken-Virus nach einem der Ansprüche 1 bis 7, dadurch gekennzeichnet, daß die DNA-Sequenz für einen Faktor codiert, der in den Geflügel-Stoffwechsel eingreift.

13. Verfahren zur Herstellung eines rekombinanten Virus nach einem der Ansprüche 1 bis 12, dadurch gekennzeichnet, daß man eine Kultur von kompetenten Zellen mit der DNA eines Plasmids transformiert, das mindestens ein heterologes Gen im Geflügelpocken-Virus aufweist, unter der Kontrolle eines Geflügelpocken-Virus-Promotors, insbesondere des Promotors des Proteins 7,5 K des Vaccine-Virus, beiderseits flankiert von dem 3'-Ende von ORF 7 und dem 5'-Ende von ORF 9, wobei die genannte Kultur vorher mit einem Geflügelpocken-Virus infiziert worden ist.

16

**14.** Verfahren zur Herstellung einer Kultur von Eukarioten-Zellen, die mit einem rekombinanten Geflügelpocken-Virus infiziert ist, dadurch gekennzeichnet, daß man die bei dem Verfahren nach einem der Ansprüche 1 bis 13 erhaltene Kultur gewinnt (abtrennt).

**15.** Verfahren zur Herstellung einer Zellenkultur nach Anspruch 14, dadurch gekennzeichnet, daß es sich dabei um Aviär-Zellen handelt.

**16.** Verfahren zur Herstellung einer Vaccine (Impfstoffes), dadurch gekennzeichnet, daß man ein nach dem Verfahren nach einem der Ansprüche 1 bis 13 erhaltenes Geflügelpocken-Virus mit einem pharmazeutisch akzeptablen Träger mischt.

**17.** Verfahren zur Herstellung einer Vaccine (Impfstoffes) nach Anspruch 16, dadurch gekennzeichnet, daß es ein Geflügelpocken-Virus enthält, das durch Replikation in den Zellenkulturen, die nach einem der Ansprüche 14 und 15 hergestellt worden sind, erhalten wurde, und daß das genannte Geflügelpocken-Virus ein lebends Virus ist.

**18.** Verfahren zur Herstellung eines Antiserums, dadurch gekennzeichnet, daß man Antikörper, die gegen das Geflügelpocken-Virus gerichtet sind, das nach einem der Ansprüche 1 bis 13 hergestellt wurde, gewinnt (abtrennt).

**19.** Verfahren zur Herstellung eines industriell oder therapeutisch interessanten Proteins, dadurch gekennzeichnet, daß man eine Eukarioten-Zelle nach den Ansprüchen 14 oder 15 züchtet und das erhaltene Protein gewinnt (abtrennt).

FIG_1

EP 0 314 569 B1

FIG_2

FIG_3

FIG_4